# EUROPEAN PATENT APPLICATION

(11) **EP 1 374 907 A2**
(43) Date of publication of application: **02.01.2004**
(21) Application number: 02017756.4
(22) Date of filing: 09.08.2002
(51) Int. Cl.: A61K 47/48

(54) **Drug transport and delivery system**

(30) Priority: 24.06.2002 EP 02013916
(71) Applicant: Ghanem, Ghanem Elias, c/o Institut Jules Bordet, 1000 Bruxelles (BE); Mehlem, Francesco, 3048 Worblaufen (CH)
(72) Inventor: Ghanem, Ghanem Elias, c/o Institut Jules Bordet, 1000 Bruxelles (BE); Mehlem, Francesco, 3048 Worblaufen (CH)
(74) Representative: Blum, Rudolf Emil Ernst

(57) **Abstract**

Described are tripeptides or tetrapeptides comprising a proteolytic enzyme cleavable amino acid moiety as a drug or pharmacologically active site or pharmacologically active group binding, transport by blood cells and delivery system as well as to the transport and delivery system coupled drugs. Such transport and delivery system is especially favorable for the treatment of diseases that lead to an enhanced activity of proteolytic enzymes such as cancer, arthritis, or are blood cell related.

## Description

The present invention concerns peptides that are able to efficiently bind to blood cells, that may act as prodrugs and as a drug delivery system, through a proteolytic cleavage and/or activation at a specific targeted site, as well as to drug bound peptides or peptide bound drugs, respectively.

Many diseases can not or not well be treated since suitable drugs cannot be administered in sufficient amounts to be effective at the site of interest due to non-specific delivery to said sites and undesired side effects at other sites. This is of particular pertinence in cancer chemotherapy.

Therefore, a transport and delivery system that ensures that a drug is more concentrated at the site of interest is of extreme value.

Hence, it is a general object of the invention to provide a transport and delivery system for pharmacologically active molecules or pharmacologically active groups, pharmacologically active molecules or pharmacologically active groups coupled to such transport and delivery system, compositions comprising them, and their suitability for the treatment of diseases.

Now, in order to implement these and still further objects of the invention, which will become more readily apparent as the description proceeds, the drug transport and delivery system of the present invention is manifested by the features that it is a tripeptide or tetrapeptide or an alkyl ester thereof comprising a proteolytic enzyme cleavable amino acid moiety as a drug or pharmacologically active site or pharmacologically active group transport and delivery system, in particular a not terminal proteolytic enzyme cleavable amino acid moiety, such as an optionally substituted phenylalanyl moiety.

Further aspects of the present invention are
- the use of a tripeptide or tetrapeptide as defined above as substituent or part of a substituent of a drug, in particular a drug for the treatment of arthritis, invasive parasitic diseases, Paludism (Malaria), AIDS, and tumours, especially cancer,
- a tripeptide or a tetrapeptide of the present invention that is connected to a drug or a pharmacologically active site or a pharmacologically active group,
- the use of such a coupled tripeptide or tetrapeptide for the preparation of a medicament for the treatment of tumors, in particular cancer, but also arthritis, invasive parasitic diseases, Paludism (Malaria), and AIDS,
- a method for improving the efficiency of a drug and/or for reducing the side effects of a drug wherein said drug is coupled to or included in a transport system as defined above,
- a pharmaceutical composition comprising a drug or pharmacologically active site or pharmacologically active group coupled tripeptide or tetrapeptide, and
- a method for the production of an active ingredient of a medicament comprising a transport and delivery system, wherein a drug or a pharmacologically active site or a pharmacologically active group is coupled to amino acids such that a tripeptide or a tetrapeptide as defined above connected to a drug or a pharmacologically active site or a pharmacologically active group is generated, with the proviso that the pharmacologically active group is not -N(CH₂-CH₂-Cl)₂.

In connection with the present invention, the terms drug, effective substance, active ingredient, effective ingredient or active substance are applied synonymously.

The present invention also includes systems where part of a substance known to be a drug is also part of the transport and delivery system. This can be the case when the drug comprises itself a proteolytic enzyme cleavable amino acid. Thus, if the transport and delivery system is drug loaded, then the cleavable amino acid, in particular the pharmacologically active group or site substituted phenylalanine moiety, can not only be substituted by the drug but be the drug. In such cases, but also if only part of a drug is needed to get the desired pharmaceutical effect, the transport and delivery system is described as coupled to or carrying a pharmacologically active site or a pharmacologically active group.

Since the drug shall become effective at sites of high proteolytic enzyme activity, the provision that the peptides of the present invention comprise at least one proteolytic enzymes cleavable site being or carrying the drug, pharmacologically active site or pharmacologically active group ensures that the pharmacologically active molecule is effectively liberated at the desired site.

Due to the specific structure of the peptides of the present invention, they are able to rapidly (within minutes) and totally bind to blood cells (mostly red blood cells; blood contains a mean of 1000 times more red than white cells per volume unit) such that said blood cells are used as carriers for the drug loaded peptides. Thereby the blood cells are used to specifically deliver drugs to sites in the body where a substantial proteolytic activity takes place. Such sites are e.g. sites with arthritis, tumors, invasive parasitic diseases etc. At such sites - due to their cleavability by proteolytic enzymes - the blood cells are deloaded with great preference.

The tripeptides or tetrapeptides of the present invention may be alkyl esters. Preferred alkyl groups are methyl and ethyl groups, much preferred the ethyl group.

Preferred optionally substituted tripeptides and tetrapeptides that can be used as transport and delivery system of the present invention comprise as the (preferably not terminal) cleavable group an optionally substituted Phe or other cleavable amino acid moiety such as Arg. A selection of possible tripeptides and tetrapeptides comprises Phe-Phe-Pro, Pro-Phe-Phe, Phe-Phe-Ser, Ser-Phe-Phe, Phe-Phe-Asn, Asn-Phe-Phe, Phe-Gly-Phe-Val (Seq. Id. No. 1), Val-Phe-Gly-Phe (Seq. Id. No. 2), Phe-Arg-Phe-His (Seq. Id. No. 3), His-Phe-Arg-Phe (Seq. Id. No. 4), Phe-Arg-Val, Val-Arg-Phe, or their lower alkyl esters.

In a preferred embodiment, the tripeptide or tetrapeptide comprises a terminal Phe that is fluoro substituted in para position. Such a preferred peptide is Pro-Phe-p-F-Phe, used in the Examples.

For easier coupling with a drug or a pharmacologically active site or a pharmacologically active group, the proteolytic enzyme cleavable amino acid moiety is substituted with a substituent sufficiently reactive to be useful in drug coupling reactions. A suitable peptide of this type, namely Pro-m-sarcolysyl-p-fluoro-Phe, is already known, however not as a transport and delivery system.

Preferred peptides are those that rapidly and totally bind to the blood cells. The rapid and total binding of such peptides to blood cells, in particular red blood cells (RBC), and their proteolytic cleavability allows to deliver drugs to the inside of blood cells but also to other cells, in particular cells with higher proteolytic activity than it is found on blood cells and other normal tissues.

The rapid association and proteolytic delivery of different molecules to the plasma may serve as e.g. cell to cell delivery system, namely in that, once bound to blood cells, the relatively weak but present cell membrane associated proteases ensure the slow generation of the active drug which can then act first on neighbouring cells in the blood.

The mechanism underlying this effect is assumed to include several steps and may be summarized as follows, whereby it is emphasized that this attempt to reason the mechanism shall by no means limit the scope of the present invention:

A first step is a complete and rapid binding, within a couple of minutes, of the peptide to the surface of blood cells. This binding is most probably due to two mechanisms of which, dependent on the type of peptide one or the other may be much preferred:
1) a specific binding to proteases, mainly metalloproteinases active sites present on the blood cell membrane and
2) a moderate non specific binding to the cell membrane because of the hydrophobic nature of the molecule greatly enhanced by alkyl ester residues, in particular the ethyl ester residue.

For the first binding mechanism the mere presence of a proteinase cleavable group is sufficient, whereby the second mechanism can be influenced by the presence of specific amino acids containing hydrophobic moieties such as Phe, Tyr, Asp, Cys, Glu, Leu, Ileu, Met, Pro, Trp, Val, etc. and the ester form.

A subsequent catalysed proteolytic reaction takes place at the blood cell site and also at any other proteolytic enzyme rich site of the body. Due to the great difference in proteolytic activity of blood cells (that can be considered as proteolytic activity poor sites) and proteolytic activity rich sites, the blood cell transported molecule is preferentially delivered to the site where proteolytic catalysis is the most active.

Since the mean circulatory time is about 12 seconds, within a time-window of up to about 15 minutes, most of the blood cells carrying the drug loaded transport system are able to reach all the organs of the body that can comprise a tumour.

Since the half-life of the blood cell-catalyzed drug generation has been determined to be at least about 20 minutes, sufficient blood cell associated drug can be available for catalysis and accumulation in the targeted area.

In the targeted area defined above, a high concentration gradient of soluble and/or membrane bound proteolytic enzymes may be found. Both the nature of these enzymes and their amount ensure the liberation of the transported molecule.

This type of delivery by proteolytic enzymes does not take place or only to a minor extent in the majority of normal organs due to their low protease expression.

One example for a very suitable application of the inventive peptides is cancer therapy. In this particular area, on the one hand the tumors often enhance the number of blood vessels and sequestrate red blood cells, on the other hand proteolytic enzymes play a crucial role to promote invasion and subsequent cancer cell spread and metastases such that numerous tumor cells do very significantly overexpress and release such proteolytic enzymes, particularly those belonging to the metal-loproteinase family. These facts promote the transport of the inventive peptides to the desired sites of activity and the drug delivery to said sites.

To this end, recent strategies focused on the design of specific inhibitors of these enzymes to prevent or delay the invasion and metastatic spread. These inhibitors are now at various stages of clinical development. By the transport and delivery system of the present invention e.g. such inhibitors can now readily and preferentially be transported to the sites of interest such that the most critical since most proteolytic enzyme producing tumors are preferentially supplied with the drug, the total amount to be administered can be reduced, and the presence of free drug in the blood stream can be lowered such that almost no free drug is in circulation.

By the transport and delivery system of the present invention, however also cancer drugs with other mechanisms of action can be supplied to the target site, or other kinds of drugs such as anti-arthritis drugs, anti-inflammatory drugs etc.

Another application of the transport and delivery system of the present invention is the targeting of blood cells themselves. The mechanism used for transport of the transport and delivery system of this invention can be used to dramatically increase the binding of any drug to blood cells. Thus the transport and delivery system of the present invention is also suitable for the treatment of numerous further diseases where the target is blood. Such diseases are e.g. Paludism (Malaria) and AIDS.

Thus, further aspects of the present invention comprise the use of tripeptides or tetrapeptides of the present invention as substituent or part of a substituent of a drug, in particular a drug for the treatment of arthritis, invasive parasitic diseases, Paludism (Malaria), AIDS, and tumors, especially cancer, the use of tripeptides and tetrapeptides of the present invention coupled to drugs or pharmacologically active sites or pharmacologically active groups as medicaments and in pharmaceutical compositions.

The present invention is now further described by means of two examples of anticancer agents, described in the pertinent art as having completely different mechanisms of action:

### 1^{st} example: PSF = Prolyl-m-sarcolysyl-p-fluoro-phenylalanin of Formula (I)

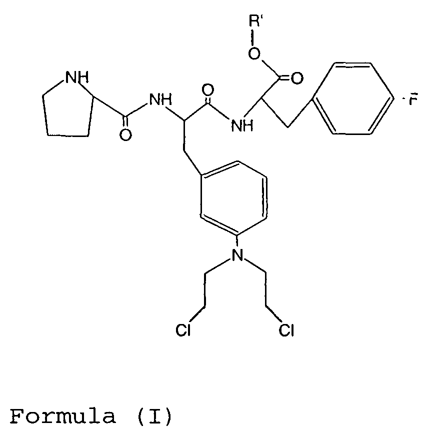

This particular substituted tripeptide is bearing an alkylating group at the sarcolysine moeity. Said alkylating group is assumed to be the pharmacologically active part of the such substituted phenylalanin moiety or - in other words - the drug loaded on the phenylalanine moiety. Although presently the ethyl ester is preferred (R' = ethyl), R' can also be other alkyl, such as methyl, or H.

Different catalytic mechanisms yielded in one major metabolite, namely m-L-sarcolysin, in presence of red blood cells and 4 metabolites in the presence of cancer cells, in this example human melanoma cells. These metabolites were identified by HPLC and mass spectrometry to be m-L-Sarcolysin, prolyl-m-L-sarcolysin, fluoro-phenylalanine and prolyl-m-sarcolysyl-p-fluoro-phenylalanin.

### 2^{nd} example: PFPP-ADM = Prolyl-adriamycin-linked-phenylalanyl-p-fluoro-phenylalanyl of Formula(II).

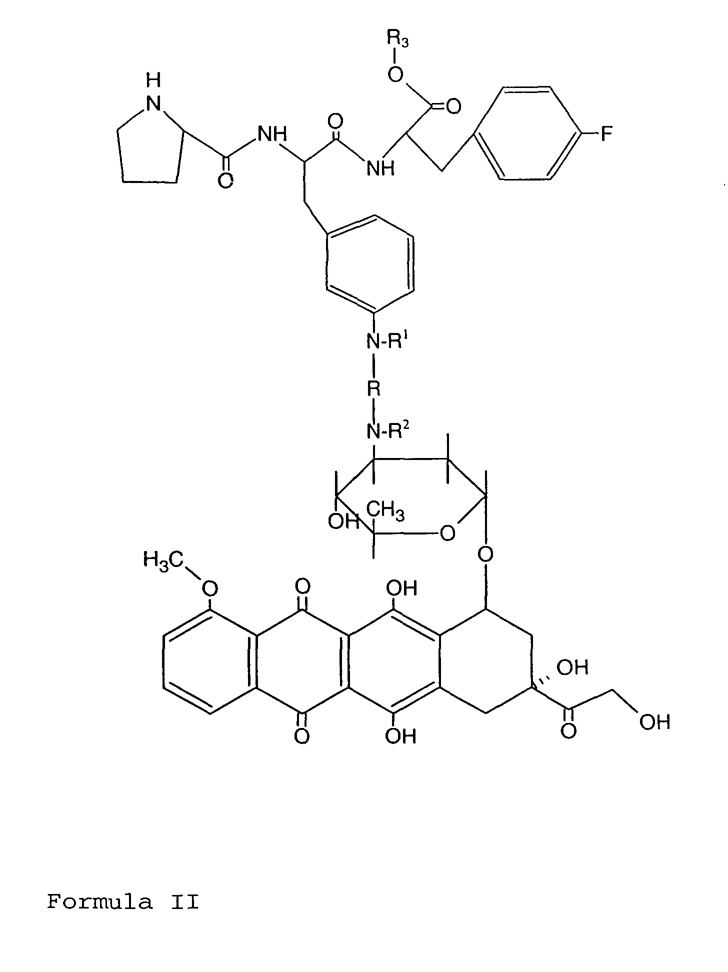

The adriamycin (doxorubicin, ADM) moiety is coupled to the N-comprising group substituted not terminal phenylalanine via its amino group by at least one hydrocarbon chain such that a connecting group -NR¹-R-NR²-is formed, wherein R is an alkylene group such as e.g. -CH₂-CH₂-, and R¹ and R² independently from each other represent H, methyl or ethyl, or R¹ and R² together form an alkylene group, such as e.g. -CH₂-CH₂-. The tripeptide can be present as acid (R₃ = H) or alkyl ester (R₃ preferably CH₃-, CH₃CH₂-).

In the presence of red blood cells no HPLC detectable metabolite could be measured while in the presence of human melanoma cells one major single metabolite could be generated. According to hitherto available results this metabolite is most probably ADM linked to one Phe moiety. This was done with 1 ml of blood containing 20 µg/ml of the ADM derivative as well as 5x10⁹ RBC compared to only 5x10⁶ melanoma cells.

### Results:

A series of experiments was run that for both drug loaded delivery systems led to similar results summarized below:
- A very rapid association of the drug loaded transport and delivery system to blood cells mainly red blood cells.
- A slow proteolytic catalysis at red blood cell membranes mainly due to metalloproteinases.
- A much quicker and more efficient proteolytic catalysis at both tumor cell membranes and by proteolytic enzymes shed by the tumor (within tumor vessels too).
- A proteolytic competition largely favouring metabolite production at the tumor site rather than in the blood.
- This proteolytic catalysis yields high amounts of active metabolites.
- The addition of RBC preloaded with drug loaded transport and delivery system (after 10 min of contact between RBC and each of the drug loaded transport and delivery system) to melanoma cells resulted in a significant generation of the above described active metabolites from the RBC.

While there are shown and described presently preferred embodiments of the invention, it is to be distinctly understood that the invention is not limited thereto but may be otherwise variously embodied and practised within the scope of the following claims.

SEQUENCE LISTING

<110>  Mehlem, Francesco
       Ghanem, Ghanem Elias

<120>  Drug transport system

<130>  06006EP1

<150>  EP 02013916.8
<151>  2002-06-24

<160>  4

<170>  PatentIn version 3.1

<210>  1
<211>  4
<212>  PRT
<213>  Artificial Sequence

<220>
<223>  Artifical peptide

<400>  1

Phe Gly Phe Val
1


<210>  2
<211>  4
<212>  PRT
<213>  Artificial Sequence

<220>
<223>  Artificial peptide

<400>  2

Val Phe Gly Phe
1


<210>  3
<211>  4
<212>  PRT
<213>  Artificial Sequence

<220>
<223>  Artificial peptide

<400>  3

Phe Arg Phe His
1


<210>  4
<211>  4
<212>  PRT
<213>  Artificial Sequence

<220>
<223>  Artificial peptide

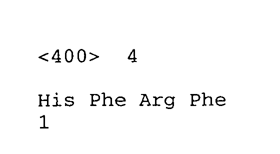

## Claims

1. A tripeptide or tetrapeptide or an alkyl ester thereof comprising a proteolytic enzyme cleavable amino acid moiety as a drug or pharmacologically active site or pharmacologically active group transport and delivery system.

2. The tripeptide or tetrapeptide of claim 1, which is an alkyl ester with the alkyl group being a methyl or an ethyl group, preferably an ethyl group.

3. The tripeptide or tetrapeptide of claim 1 or 2, wherein the proteolytic enzyme cleavable amino acid moiety is a not terminal moiety.

4. The tripeptide or tetrapeptide of anyone of the preceding claims comprising a not terminal optionally substituted phenylalanyl moiety.

5. The tripeptide or tetrapeptide of anyone of the preceding claims that is selected from the group consisting of substituted or unsubstituted Phe-Phe-Pro, Pro-Phe-Phe, Phe-Phe-Ser, Ser-Phe-Phe, Phe-Phe-Asn, Asn-Phe-Phe, Phe-Gly-Phe-Val (Seq. Id. No. 1), Val-Phe-Gly-Phe (Seq. Id. No. 2), Phe-Arg-Phe-His (Seq. Id. No. 3), His-Phe-Arg-Phe (Seq. Id. No. 4), Phe-Arg-Val, Val-Arg-Phe, whereby Pro-Phe-Phe is preferred.

6. The tripeptide or tetrapeptide of anyone of the preceding claims, wherein the terminal Phe is fluoro substituted in para position, in particular the peptide Pro-Phe-p-F-Phe.

7. A tripeptide or tetrapeptide wherein the proteolytic enzyme cleavable amino acid moiety is substituted with a substituent sufficiently reactive to be useful in drug coupling reactions, with the proviso that said substituent is not -N(CH₂-CH₂-Cl)₂ in meta position on the not terminal Phe of Pro-Phe-p-F-Phe.

8. The tripeptide or tetrapeptide of claim 7 wherein the proteolytic enzyme cleavable amino acid moiety is or comprises Phe.

9. Use of a tripeptide or tetrapeptide as defined in anyone of the preceding claims as substituent or part of a substituent of a drug, in particular a drug for the treatment of, arthritis, invasive parasitic diseases, Paludism (Malaria), AIDS, and tumours, especially cancer.

10. A tripeptide or a tetrapeptide as defined in anyone of claims 1 to 8 that is connected to a drug or a pharmacologically active site or a pharmacologically active group, with the proviso that it is not prolyl-m-sarcolysyl-p-fluoro-phenylalanine.

11. The tripeptide or tetrapeptide of claim 10 wherein the drug is adriamycin.

12. Use of the tripeptide or tetrapeptide of claim 10 or 11 for the preparation of a medicament for the treatment of cancer.

13. Use of a tripeptide or tetrapeptide as defined in anyone of claims 1 to 8 that is connected to a drug or a pharmacologically active site or a pharmacologically active group for the preparation of a medicament for the treatment of arthritis, non cancerous tumours, invasive parasitic diseases, Paludism (Malaria), and AIDS.

14. A method for improving the efficiency of a drug and/or for reducing the side effects of a drug wherein said drug is coupled to or included in a transport system of one of claims 1 to 8.

15. Use of a drug of claim 10 or 11 for the preparation of a medicament.

16. A pharmaceutical composition comprising a tripeptide or a tetrapeptide of claim 10 or 11.

17. Method for the production of an active ingredient of a medicament comprising a transport and delivery system, wherein a drug or a pharmacologically active site or a pharmacologically active group is coupled with amino acids such that a tripeptide or a tetrapeptide as defined in one of claims 1 to 7 connected to a drug or a pharmacologically active site or a pharmacologically active group is generated, with the proviso that the pharmacologically active group is not -N(CH₂-CH₂-Cl)₂.
